# EUROPEAN PATENT SPECIFICATION

(11) **EP 3 261 517 B1**
(45) Date of publication and mention of the grant of the patent: **26.12.2018**
(21) Application number: 16708743.6
(22) Date of filing: 28.01.2016
(51) Int. Cl.: A61B 1/31, A61B 1/00

(54) **A MEDICAL DEVICE FOR COLONOSCOPY**
MEDIZINISCHE VORRICHTUNG FÜR KOLONOSKOPIE
DISPOSITIF MÉDICAL POUR COLOSCOPIE

(30) Priority: 24.02.2015 TR 201502185
(43) Date of publication of application: 03.01.2018
(73) Proprietor: Bogazici Universitesi, Bebek Istanbul (TR)
(72) Inventor: SAMUR, Evren, 34342 Istanbul (TR); YARBASI, Efe Yamac, 34342 Istanbul (TR); OZAN, Berzah, Atasehir/Istanbul (TR)
(74) Representative: Dericioglu Kurt, Ekin
(86) International application number: PCT/TR2016/050022
(87) International publication number: WO 2016/137417

(56) References cited:
- WO-A2-2008/099389
- WO-A2-2009/050668
- JP-A- 2006 141 935
- US-A1- 2008 091 063

## Description

### Field of the Invention

The present invention relates to a device developed for flexible endoscopy applications with colonoscopy purposes in medical sector.

### Background of the Invention

Various endoscopes of flexible and nonflexible types are used especially for imaging in stomach and colon in medical sector. Due to the anatomical structure of the colon, especially in turns in "Sigmoid Colon" area, since methods of pushing the endoscope and turning the endoscope tip with mechanical components are used, the doctors performing the operation have difficulty during colonoscopy applications. This difficulty can cause the patients to feel pain, and in case of more stress it may cause perforation in colon walls.

In some embodiments known in the state of the art, the endoscope tip can be rotated where it is present by temporarily fixing the position of the tip by means of balloons, and then moving the endoscope is performed by the doctor/surgeon by pushing with hand from outside (manually). However, this conventional method performed manually causes pain in patients and medical problems such as perforation in the colon at corners especially where the endoscope turns, and also causes difficulties such as not being able turn completely and eye-hand coordination problems of doctors.

An international patent application numbered WO2008099389A2 discloses A self-propelled device for locomotion through a lumen, comprising a set of serially arranged inflatable chambers, with a distal chamber at one end of said device, and a proximal chamber at the opposite end of said device; a fluid supply system for inflating said chambers sequentially, such that said device moves with the distal chamber leading; and an axial channel running axially through said device, said channel being attached to a distal part of said device.

A United States patent application numbered US20080091063A1 discloses a locomotive endoscope assembly including a locomotive endoscope head, including a main portion extending along a longitudinal axis and having a first selectably inflatable balloon associated therewith and a selectably positionable portion, selectably axially positionable along the main portion and having a second selectably inflatable balloon associated therewith and a locomotive endoscope head controller controlling the operation of the locomotive endoscope head and being operative for controlling positioning of the selectably positionable portion relative to the main portion and selectable inflation of the first and second selectably inflatable balloons.

A Japanese patent application numbered JP2006141935A discloses an automatic inserting machine for pressure valve balloon expansion type endoscope.

### The Problems Solved with the Invention

The objective of the present invention is to provide a medical device which enables the endoscope tip to move inside the colon on its own via a pneumatic system. By this means, difficulties such as time loss experienced during turns, medical problems in patients such as pain and perforation due to the high mechanical forces applied on the colon walls upon manual push of the endoscope tip by the doctor performing the operation, not being able to take turns completely, and not providing eye-hand coordination of the doctor are eliminated.

The problems experienced in the conventional manually-performed methods are solved in the suggested invention by means of a chamber made inside an endoscope tip connected to a shaft and at least one balloon coming out of the said chamber by being inflated, push of the said endoscope tip upon inflation of the said balloon/balloons and a flexible shaft moving forward during this push and taking the form of the colon. In another embodiment of the invention, a different solution for the same technical problem is provided by enabling the endoscope tip to move forward and to be guided without using a chamber in the endoscope tip.

### Detailed Description of the Invention

The medical device developed to fulfill the objectives of the present invention is illustrated in the accompanying figures, in which:
Figure 1 is the side view of the inventive medical device wherein the balloons therein are not inflated
Figure 2 is the side view of the inventive medical device wherein the balloons therein are inflated.
Figure 3 is the cross sectional view of the inventive medical device wherein the balloons therein are inflated.
Figure 4 is the front view of the part of the shaft connected to the endoscope tip.

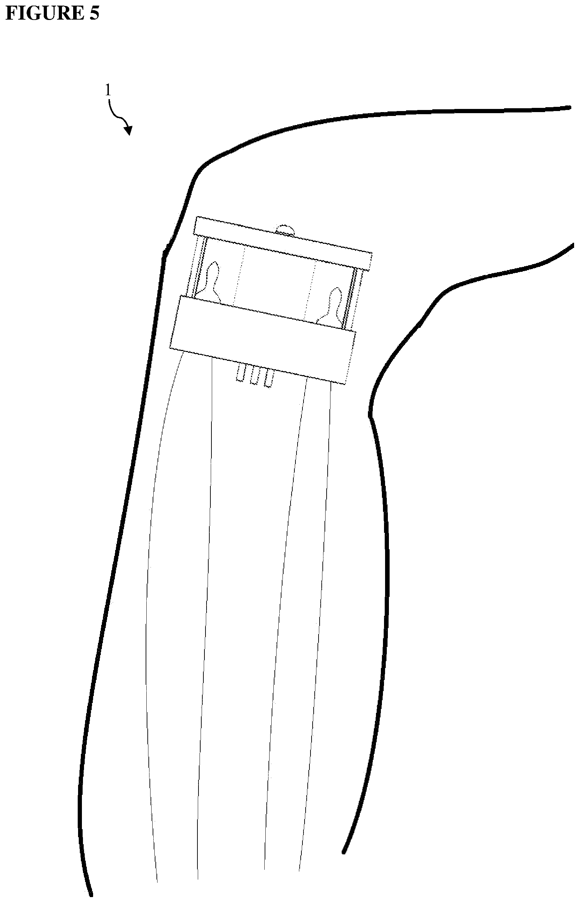

Figure 5 is the view of the medical device in a colon.
Figure 6 is another view of the medical device in a colon.
Figure 7 is yet another view of the medical device in a colon.
Figure 8 is a further view of the medical device in a colon.
Figure 9 is another view of the medical device in a colon.
Figure 10 is a further view of the medical device in a colon.
Figure 11 is a view of the medical device in Caecum area in a colon.
Figure 12 is the lateral view of the structure used in one embodiment of the inventive medical device in which an uninflated balloon is stored.
Figure 13 is the side view of the inventive medical device in an embodiment wherein the first chamber is not used.

The components shown in the figures are each given reference numbers as follows:
**1.** Medical device
**2.** Shaft
**3.** Endoscope tip
   **3.1.** Rear wall
   **3.2.** Hole
   **3.3.** Front wall
**4.** First chamber
**5.** Balloon
   **5.1.** Closed tip
   **5.2.** Inlet
   **5.3.** Inflated part
   **5.4.** Deflated part
**6.** Air chamber
**7.** Cable conduit
**8.** Retraction mechanism
   **8.1.** Shaft
   **8.2.** Spiral spring
**D.** Hardware

A medical device, which can be moved via a pneumatic system for endoscopic applications, essentially comprises
- at least one shaft (2) which is manufactured from a flexible material and which can elongate,
- at least one endoscope tip (3) which is mounted on one end of the shaft (2),
- at least one first chamber (4) comprising a rear wall (3.1) and a front wall (3.2), whereby the first chamber is provided inside the endoscope tip (3),
- at least one cylindrical type balloon (5) which can elongate in the axial direction through the colon as it is inflated, which has an inlet (5.2) and a closed tip (5.1), of which the inflation part which is the inlet (5.2) is connected to one end of the shaft (2), the inflated part (5.3) of which is inside the shaft (2) and the remaining deflated part (5.4) is inside the first chamber (4), and wherein the balloon (5) enables the endoscope tip (3) to be advanced by pressing on the rear wall (3.1) of the endoscope tip (3) by its inflated part (5.3) inside the shaft (2) during its inflation.

The inventive medical device (1) is essentially comprised of two main parts. The said parts are and elongating flexible shaft (2) and an endoscope tip (3) that can be mounted at the end of this shaft (2). The primary principle of this embodiment of the invention is to create a force on the endoscope tip (3) by inflating one or more than one cylindrical balloons (5) placed inside the first chamber (4) the closed tips (5.1) of which are present within the endoscope tip (3) and preferably being able to elongate in an axial direction as it inflates in equal or different sizes, and to move the endoscope tip (3) by guiding it. Therefore, it is enabled that various hardware (D) attached to a hole/holes (3.2), located in the endoscope tip (3) and enabling to perform medical processes (biopsy, lighting, imaging, cleaning, gas transfer, etc.), are guided and moved. It is explained in detail with various applications below how the balloon (5) is placed inside the first chamber (4) and how the endoscope tip (3) is guided and moved by inflating the balloons (5).

In a preferred embodiment of the invention, there is preferably a small opening in the endoscope tip (3), in the part wherein the endoscope tip (3) is connected to the shaft (2). In this opening, the inlet (5.2) of a balloon (5) is placed to the shaft (2). In another embodiment of the invention, the inlet (5.2) of the balloon (5) is connected to the end of the shaft (2) outside the patient. All of the part of the said balloon (5) apart from the inlet (5.2) is stored in the first chamber (4) in a slightly stretched form. When air is pumped in by means of a device from one end, which is not connected to the balloon (5), of the shaft (2), through which air can flow in a sealed way, the air enters from the inlet (5.2) of the balloon (5) attached to the shaft (2). Therefore, the balloon (5), of which the closed tip (5.1) and the deflated part (5.4) are inside the first chamber (4), is inflated linearly, and the endoscope tip (3) is enabled to move forward as a result of the force created on the rear wall (3.1) of the endoscope tip (3) by means of its inflated part (5.3). The endoscope tip (3) pulls the flexible shaft (2) and the various hardware (D) enabling to perform medical procedures (for example imaging with fiber optic camera), which are connected to itself, during its forward movement. In one embodiment of the invention, the endoscope tip (3) has a structure which can easily be attached to and removed from the elongating flexible shaft (2). One end of the endoscope tip (3) and the shaft (3) are in form of a telescopic circle, and has a lock mechanism in form of push and turn as it is in medicine caps. Therefore, after being used, deformed balloons (5) can easily be changed between operations. Again in this embodiment of the invention, there are two air chambers (6) which are present in the shaft (2) and normally left empty. These air chambers (6) can be positioned within the shaft (2) preferably on an axis vertically intersecting the axis where the balloons are located such that there will be a predetermined distance in between them. The said air chambers (6) are inflated and deflated by a device through the one end of the shaft (2), which is outside the patient and the air chambers (6) filled with air, are able to expand outwards. Therefore, it allows the shaft (2) to be moved in the axis that cannot be affected by the balloons (5) and the angular range that can be viewed via a various hardware (D) (for example a fiber optic camera) enabling to perform medical procedures to be increased too.

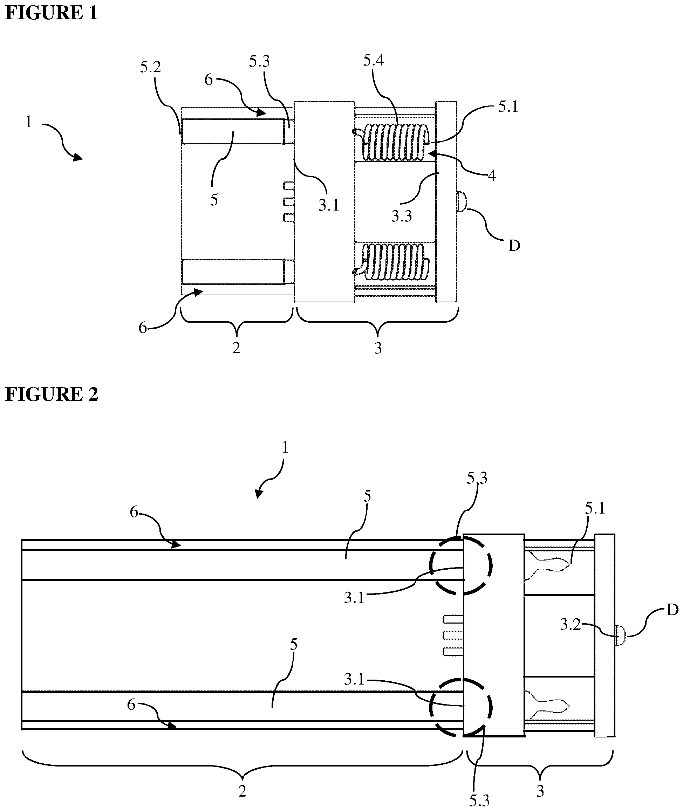

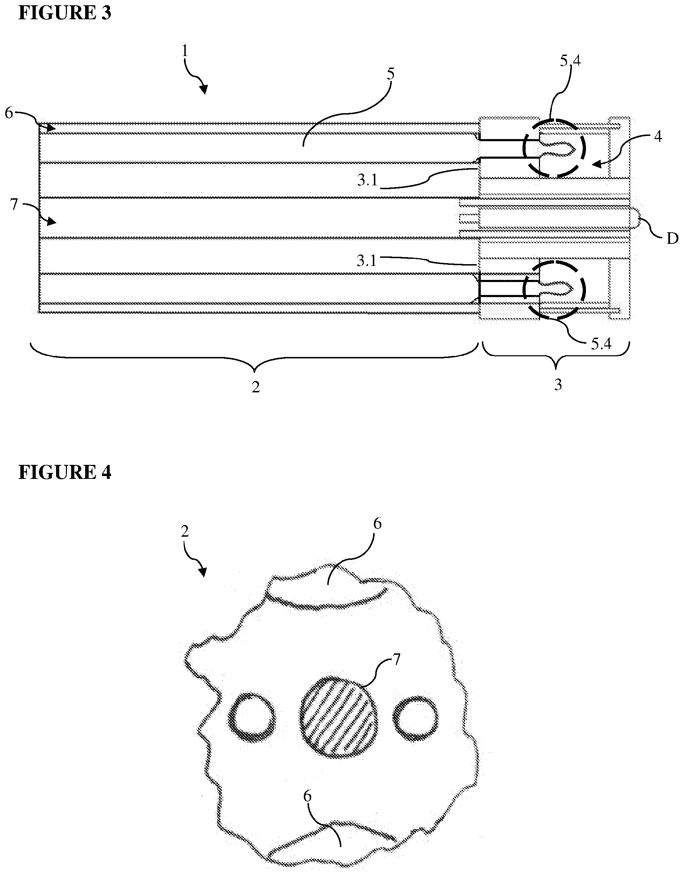

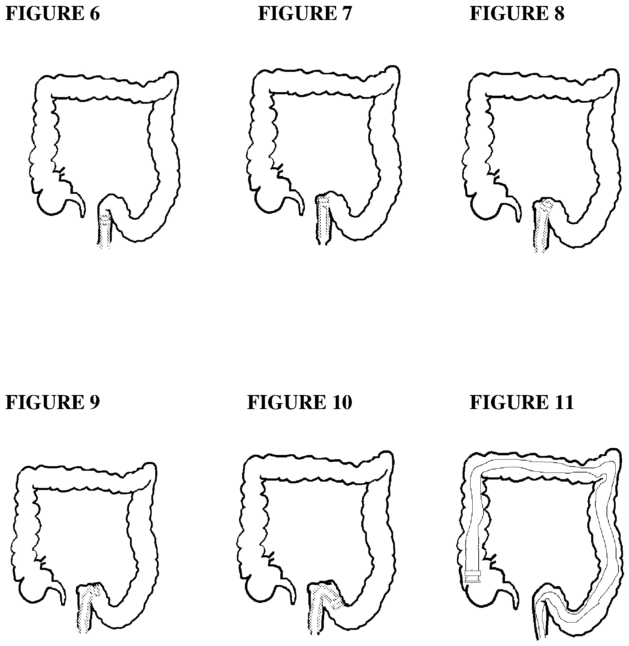

In another preferred embodiment of the invention, two balloons (5) are used. The inlets (5.2) of the two balloons (5) in the said opening inside the endoscope tip (3) are placed to the shaft (2) such that there will be a predetermined distance in between them. In another embodiment of the invention, the inlets (5.2) of the balloons (5) are connected to the end of the shaft (2) outside the patient. When the balloons (5) are inflated by giving air by a device through the end of the shaft (2) outside the patient, the endoscope tip (3) is moved forward by pressing on the rear wall (3.1) of the endoscope tip (3) via the said balloons (5) (Figure 2). When the balloons (5) are inflated up to different sizes, according to the positions of the inflated parts (5.3) of the balloons (5.3), different amounts of force is applied on two different points of the rear wall (3.1) of the endoscope tip (3). Therefore, according to the axis where the balloons (5) are positioned, the angular position of the endoscope tip (3) in the horizontal or vertical axis can be changed as it will be right/left or up/down, and the endoscope tip (3) is enabled to move forward (Figures 8, 9, and 10). Again in this embodiment of the invention, there are two air chambers (6) which are present in the shaft (2) and normally left empty (Figure 4). These air chambers (6) can be positioned within the shaft (2) preferably on an axis vertically intersecting the axis where the balloons are located such that there will be a predetermined distance in between them. The said air chambers (6) are inflated by a device through the one end of the shaft (2), which is outside the patient, and the air chambers (6) filled with air are enabled to expand outwards. Therefore, the shaft (2) is enabled to move in the axis that cannot be affected by the balloons (5) and the angular distance that can be imaged via various hardware (D) enabling to perform medical procedures is enabled to be increased.

In another embodiment of the invention, the said balloons (5) are not present in a single first chamber (4), they are separately present in at least two independent first chambers (4). Furthermore, for the hardware (D) placed at the endoscope tip (3) for endoscopic applications, there can be a separate chamber preferably in the middle part inside the endoscope tip (3). Furthermore, in order to provide a cable communication with the said hardware (D), there is a cable conduit (7) extending from the central axis of the shaft (2), from one end of the shaft (2) outside the patient towards the endoscope tip (3).

In another preferred embodiment of the invention, four balloons (5) are used. The inlets (5.2) of the four balloons (5) in the said opening inside the endoscope tip (3) are placed to the shaft (2) such that there will be a predetermined distance in between them on two axes preferably intersecting vertically each other. In another embodiment of the invention, the inlets (5.2) of the balloons (5) are connected to the end of the shaft (2) outside the patient. When the said balloons (5) are inflated up to different sizes by giving air through the end of the shaft (2) outside the patient via a device, according to the positions of the inflated parts (5.3) of the balloons (5.3), the angular position of the endoscope tip (3) in different axes (in other words the horizontal or vertical axis) can be changed as it will be both right/left and up/down, and the endoscope tip (3) is enabled to move forward.

In another embodiment of the invention, the said balloons (5) are not present in a single first chamber (4), they are separately present in at least four independent first chambers (4). Furthermore, for the hardware (D) placed at the endoscope tip (3) for endoscopic applications, there can be a separate chamber preferably in the middle part inside the endoscope tip (3). Furthermore, in order to provide a cable communication with the said hardware (D), there is a cable conduit (7) extending from the central axis of the shaft (2), from one end of the shaft (2) outside the patient towards the endoscope tip (3).

In one embodiment of the invention, there is a retraction mechanism (8) fixed preferably on the front wall (3.3) within the first chamber (4) so that retraction performed while deflating the balloon/balloons (5) can be performed in a more controlled way. The said retraction mechanism (8) is comprised of a shaft (8.1) preferably on which the closed tip (5.1) of the balloon (5) is fixed and around which the deflated part (5.4) of the balloon (5) is wrapped, and a spiral spring (8.2) to which the shaft (8.1) is attached in order to provide the rotary movement required for this wrapping process. While the balloon/balloons (5) are being inflated, they rotate the shaft (8.1) of the retraction mechanism (8), and thus the spiral spring (8.2) connected to the shaft (8.1) is stretched. Therefore, while the balloon/balloons (5) are deflated, it is enabled that the deflated part (5.4) of the balloon/balloons (5) are wrapped around the shaft (8.1) rotating in opposite direction by means of the spiral spring (8.2) and collected inside the first chamber (4). Therefore, it allows the deflated balloon (5) to be gathered back and the endoscope tip (3) to be retracted by pulling backwards.

By using the inventive medical device (1), for example after reaching the farthermost point in the colon (Caecum) that can be reached, the said balloon/balloons (5) are slowly deflated and the medical device (1) is retracted. During this process, medical procedures required by the surgeon (examining the colon walls, biopsy, etc.) are performed. In figure 11, the medical device (1) is shown as it reaches the Caecum.

The number of balloons (5) positioned inside the first chamber (4), as it is mentioned above, is not fixed, various configurations can be formed according to different endoscopic applications of the medical device (1) by increasing the number of balloons (5) by a person skilled in the art in order to increase the accuracy in turns.

In another preferred embodiment of the invention, the endoscope tip (3) can be moved forward and guided by means of inflating one or more than one balloons (5) without using the first chamber (4). It is explained in details below how the endoscope tip (3) is moved without using the first chamber (4).

A medical device, which can be moved via a pneumatic system for endoscopic applications, essentially comprises
- at least one shaft (2) which is manufactured from a flexible material and which can elongate,
- at least one endoscope tip (3) which is mounted on one end of the shaft (2),
- at least one cylindrical type balloon (5) which can elongate in the axial direction through the colon as it is inflated, which has an inlet (5.2) and a closed tip (5.1), of which the inflation part which is the inlet (5.2) is connected to one end of the shaft (2), and wherein the balloon (5) enables the endoscope tip (3) to be advanced by pressing on the front wall (3.3) of the endoscope tip (3) by its inflated part (5.3) during its inflation.

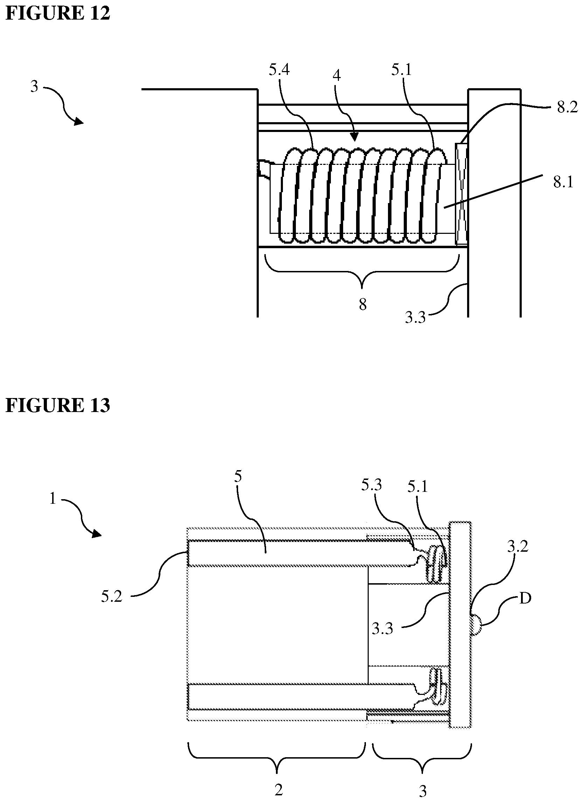

The main operating principle of this embodiment of the invention is to inflate one or more than one cylindrical balloons (5) connected to one end of the shaft and preferably being able to elongate in an axial direction as it is inflated in equal or different sizes, and to create force on the front wall (3.3) of the endoscope tip (3), and to move the endoscope tip (3) by guiding it (Figure 13). Therefore, it is enabled that various hardware (D) attached to a hole/holes (3.2) made on the endoscope tip (3) to enable performing medical procedures (biopsy, lighting, imaging, cleaning, gas transfer, etc.) are guided and moved.

In a preferred embodiment of the invention, the inlet (5.2) of the balloon (5) is connected to the end of the shaft (2) outside the patient. The whole of the part outside the inlet (5.2) of the said balloon (5) is present within the shaft (2) towards the endoscope tip (3) in a slightly stretched way. When air is pumped in through one end, which not connected to the balloon (5), of the shaft (2), through which air can flow in a sealed way, or directly from the inlet (5.2) of the balloon (5), by means of a device, the air enters from the inlet (5.2) of the balloon (5) attached to the shaft (2). Therefore, it is enabled that the balloon (5) inflates linearly and the endoscope tip (3) moves forward as a result of the force created by the inflated part (5.3) on the front wall (3.3) of the endoscope tip (3). The endoscope tip (3) pulls the flexible shaft (2) and the various hardware (D) enabling to perform medical procedures (for example imaging with fiber optic camera) connected to itself during its forward movement.

In an embodiment of the invention, there are two air chambers (6) which are present in the shaft (2) and normally left empty. These air chambers (6) can be positioned within the shaft (2) preferably on an axis vertically intersecting the axis where the balloons are located such that there will be a predetermined distance in between them. The said air chambers (6) are inflated by a device through the one end of the shaft (2), which is outside the patient, and the air chambers (6) filled with air are enabled to expand outwards. Therefore, it is enabled that the shaft (2) is moved in the axis that is not affected by the balloons (5) and the angular range that can be viewed via a various hardware (D) (for example a fiber optic camera) enabling to perform medical procedures is increased.

In another preferred embodiment of the invention, two balloons (5) are used. The inlets (5.2) of the said two balloons (5) are connected to an end of the shaft (2), which is preferably outside the patient, such that there will be a predetermined distance in between them. The endoscope tip (3) is moved by applying a force on the rear wall (3.3) of the endoscope tip (3) via the said balloons (5) when the balloons (5) are inflated by giving air through the end of the shaft (2) outside the patient or directly through the inlets (5.2) of the balloons (5) by a device. When the balloons (5) are inflated up to different sizes, according to the positions of the inflated parts (5.3) of the balloons (5.3), different amounts of pressure are applied on two different points of the front wall (3.3) of the endoscope tip (3). Therefore, according to the axis where the balloons (5) are positioned, the angular position of the endoscope tip (3) in the horizontal or vertical axis can be changed as it will be right/left or up/down, and the endoscope tip (3) is enabled to move forward. Again in this embodiment of the invention, there are two air chambers (6) which are present in the shaft (2) and normally left empty. These air chambers (6) can be positioned within the shaft (2) preferably on an axis vertically intersecting the axis where the balloons are located such that there will be a predetermined distance in between them. The said air chambers (6) are inflated by a device through the one end of the shaft (2), which is outside the patient, and the air chambers (6) filled with air are enabled to expand outwards. Therefore, it is enabled that the shaft (2) is moved in the axis that is not affected by the balloons (5) and the angular distance that can be viewed via various hardware (D) enabling to perform medical procedures is increased.

In another preferred embodiment of the invention, four balloons (5) are used. The inlets (5.2) of the balloons (5) are connected to the end of the shaft (2) outside the patient. When the said balloons (5) are inflated up to different sizes by giving air through the end of the shaft (2) outside the patient or directly through the inlets (5.2) of the balloons (5) via a device, according to the positions of the inflated parts (5.3) of the balloons (5.3), the angular position of the endoscope tip (3) in different axes (in other words the horizontal or vertical axis) can be changed as it will be both right/left and up/down, and the endoscope tip (3) is enabled to move forward.

In one embodiment of the invention, in order to provide a cable communication with the said hardware (D), there is a cable conduit (7) extending from the central axis of the shaft (2), from one end of the shaft (2) outside the patient towards the endoscope tip (3).

The number of balloons (5) positioned inside the first chamber (4), as it is mentioned above, is not fixed, various configurations can be formed according to different endoscopic applications of the medical device (1) by increasing the number of balloons (5) by a person skilled in the art in order to increase the accuracy in turns.

## Claims

1. A medical device (1), which can be moved via a pneumatic system for endoscopic applications, **comprising**
- a shaft (2),
- an endoscope tip (3) which is mounted on one end of the shaft (2),
**and characterized by**
- the shaft (2) which is manufactured from a flexible material and which can elongate,
- at least one first chamber (4) comprising a rear wall (3.1) and a front wall (3.2), whereby the first chamber is provided inside the endoscope tip (3),
- at least one cylindrical type balloon (5) which can elongate in the axial direction through the colon as it is inflated, which has an inlet (5.2) connected to one end of the shaft (2) and a closed tip (5.1) disposed in the first chamber (4), and wherein the balloon (5) enables the endoscope tip (3) to be advanced by pressing on the rear wall (3.1) of the endoscope tip (3) during its inflation.

2. The medical device (1) according to claim 1, **characterized by** the shaft (2) through which air can be passed in a sealed way when air is given through its one end not connected to the balloon (5).

3. The medical device (1) according to claim 1, **characterized by** at least one retraction mechanism (8) which is fixed preferably on the front wall (3.3) within the first chamber (4) so that retraction performed during deflating the balloon/balloons (5) can be performed in a more controlled way and comprised of a shaft (8.1) preferably on which the closed tip (5.1) of the balloon (5) is fixed and around which the deflated part (5.4) of the balloon (5) is wrapped, and a spiral spring (8.2) to which the shaft (8.1) is attached in order to provide the rotary movement required for this wrapping process.

4. The medical device (1) according to claim 1, **characterized by** a separate chamber preferably in the middle part inside the endoscope tip (3) for the hardware (D) placed at the endoscope tip (3) for endoscopic applications.

5. The medical device (1) according to claim 1, **characterized by** the at least one balloon (5) which enables the endoscope tip (3) to be advanced by pressing on the front wall (3.3) of the endoscope tip (3) during its inflation.

6. The medical device (1) according to claim 1, **characterized by** at least one hole (3.2) made on the endoscope tip (3) so that a hardware (D) enabling to perform medical procedures can be attached.

7. The medical device (1) according to claim 1, **characterized by** at least two air chambers (6) which are positioned inside the shaft (2) such that there will be a predetermined distance between them in an axis vertically intersecting the axis where the balloon (5) is positioned, and which enables the shaft (2) to be moved in an axis that cannot be affected by the balloons (5) by being inflated and deflated and enables angular distance that can be viewed via a hardware (D) enabling to perform medical procedures to be increased.

8. The medical device (1) according to claim 1, **characterized by** two balloons (5), the inlets (5.2) of which are placed to the shaft (2) such that there will be a distance between them and which enables to change the angular position of the endoscope tip (3) and to move the endoscope tip (3) forward at the same time when they are inflated to different sizes by giving air through the end of the shaft (2) outside the patient via a device.

9. The medical device (9) according to claim 1, **characterized by** air chambers (6) which are positioned inside the shaft (2) in an axis vertically intersecting the axis where the balloons (5) are positioned, and which enables the shaft (2) to be moved in an axis that cannot be affected by the balloons (5) by being inflated and deflated by a device and enables angular distance that can be viewed via a hardware (D) enabling to perform medical procedures to be increased.

10. The medical device (1) according to claim 1, **characterized by** four balloons (5) the inlets (5.2) of which are placed to the shaft (2) such that they will be on two axes vertically intersecting each other and which enables to change the angular position of the endoscope tip (3) in two different axes and to move the endoscope tip (3) forward at the same time when they are inflated to different sizes by giving air through the end of the shaft (2) outside the patient via a device.

11. The medical device (1) according to claim 1, **characterized by** a cable conduit (7) which extends from the central axis of the shaft (2) towards the endoscope tip (3) from one end of the shaft (2) outside the patient in order to provide cable communication with a hardware (D) placed to the endoscope tip (3) for endoscopic applications.

## Patentansprüche

1. Medizinische Vorrichtung (1), die über ein pneumatisches System für endoskopische Anwendungen bewegt werden kann, **umfassend**
- ein Shaft (2),
- eine Endoskopspitze (3), die an einem Ende des Shaftes (2) montiert ist,
**und gekennzeichnet durch dass**
- der Schaft (2), die aus einem flexiblen Material hergestellt ist und sich dehnen kann
- mindestens eine erste Kammer (4) mit einer Rückwand (3.1) und einer Vorderwand (3.2), worin die erste Kammer innerhalb der Endoskopspitze (3) vorgesehen ist,
- mindestens einen zylindrischen Ballon (5), der sich beim Aufblasen in axialer Richtung durch den Dickdarm dehnen kann, der einen mit einem Ende des Schaftes (2) verbundenen Einlass (5.2) und eine in der ersten Kammer (4) angeordnete geschlossene Spitze (5.1) aufweist, und wobei der Ballon (5) ermöglicht, dass die Endoskopspitze (3) durch Drücken auf die Rückwand (3.1) der Endoskopspitze (3) während des Aufblasens vorgeschoben wird.

2. Medizinische Vorrichtung (1) nach Anspruch 1, **gekennzeichnet durch dass** den Schaft (2), durch den Luft dicht geführt werden kann, wenn Luft durch ihr einziges, nicht mit dem Ballon (5) verbundenes Ende geleitet wird.

3. Medizinische Vorrichtung (1) nach Anspruch 1, **gekennzeichnet durch dass** mindestens einen Einziehmechanismus (8), der vorzugsweise an der Vorderwand (3.3) innerhalb der ersten Kammer (4) fixiert ist, so dass das Einziehen beim Entleeren des Ballons bzw. der Ballons (5) kontrollierter perfoniert werden kann und aus einem Schaft (8.1) besteht vorzugsweise an der die geschlossene Spitze (5.1) des Ballons (5) fixiert ist und um die der entleerte Teil (5.4) des Ballons (5) gewickelt ist, und eine Spiralfeder (8.2), an dem Schaft (8.1) befestigt ist, um die für diesen Wickelvorgang erforderliche Drehbewegung zu gewährleisten.

4. Medizinische Vorrichtung (1) nach Anspruch 1, **gekennzeichnet durch dass** eine separate Kammer vorzugsweise im Mittelteil innerhalb der Endoskopspitze (3) für die an der Endoskopspitze (3) platzierte Hardware (D) für endoskopische Anwendungen.

5. Medizinische Vorrichtung (1) nach Anspruch 1, **gekennzeichnet durch dass** den mindestens einen Ballon (5), der es ermöglicht, die Endoskopspitze (3) durch Drücken auf die Vorderwand (3.3) der Endoskopspitze (3) während ihres Aufblasens vorwärts zu bewegen.

6. Medizinische Vorrichtung (1) nach Anspruch 1, **gekennzeichnet durch dass** mindestens ein Loch (3.2) an der Endoskopspitze (3), so dass eine Hardware (D) zur Durchführung medizinischer Eingriffe befestigt werden kann.

7. Medizinische Vorrichtung (1) nach Anspruch 1, **gekennzeichnet durch dass** mindestens zwei Luftkammern (6), die innerhalb des Schaftes (2) so positioniert sind, dass ein vorbestimmter Abstand zwischen ihnen in einer Achse besteht, die vertikal die Achse schneidet, in der der Ballon (5) positioniert ist, und die es ermöglicht, den Schaft (2) in einer Achse zu bewegen, die durch Aufblasen und Entleeren nicht von den Ballonen (5) beeinflusst werden kann, und die einen Winkelabstand ermöglicht, der über eine Hardware (D) betrachtet werden kann, die es ermöglicht, medizinische Eingriffe durchzuführen, vergrößert zu werden.

8. Medizinische Vorrichtung (1) nach Anspruch 1, **gekennzeichnet durch dass** zwei Ballons (5), deren Einlässe (5.2) so auf den Schaft (2) aufgesetzt sind, dass ein Abstand zwischen ihnen besteht und die es ermöglichen, die Winkellage der Endoskopspitze (3) zu verändern und die Endoskopspitze (3) gleichzeitig beim Aufblasen auf verschiedene Größen vorwärts zu bewegen, indem Luft durch das Ende des Schaftes (2) außerhalb des Patienten über eine Vorrichtung zugeführt wird.

9. Medizinische Vorrichtung (9) nach Anspruch 1, **gekennzeichnet durch dass** Luftkammern (6), die innerhalb des Schaftes (2) in einer Achse positioniert sind, die die Achse, in der die Ballons (5) positioniert sind, vertikal schneidet, und die es ermöglichen, den Schaft (2) in einer Achse zu bewegen, die von den Ballons (5) nicht beeinflusst werden kann, indem sie von einer Vorrichtung aufgeblasen und entleert werden, und die einen Winkelabstand ermöglicht, der über eine Hardware (D) betrachtet werden kann, die es ermöglicht, medizinische Eingriffe durchzuführen, erhöht werden.

10. Medizinische Vorrichtung (1) nach Anspruch 1, **gekennzeichnet durch dass** vier Ballons (5), deren Einlässe (5.2) so auf dem Schaft (2) platziert sind, dass sie sich auf zwei sich vertikal schneidenden Achsen befinden und die es ermöglichen, die Winkellage der Endoskopspitze (3) in zwei verschiedenen Achsen zu verändern und die Endoskopspitze (3) gleichzeitig vorwärts zu bewegen, wenn sie auf verschiedene Größen aufgeblasen werden, indem sie Luft durch das Ende des Schafts (2) außerhalb des Patienten über eine Vorrichtung abgeben.

11. Medizinische Vorrichtung (1) nach Anspruch 1, **gekennzeichnet durch dass** eine Kabelführung (7), die sich von der Mittelachse des Schaftes (2) zur Endoskopspitze (3) von einem Ende des Schaftes (2) außerhalb des Patienten erstreckt, um eine Kabelverbindung mit einer an der Endoskopspitze (3) platzierten Hardware (D) für endoskopische Anwendungen herzustellen..

## Revendications

1. Dispositif médical (1), qui peut être déplacé via un système pneumatique pour des applications endoscopiques, **comprenant**
- un arbre (2),
- une pointe (3) de l'endoscope qui est montée sur une extrémité de l'arbre (2),
**et caractérisé par**
- l'arbre (2) qui est fabriqué en un matériau flexible et qui peut s'allonger,
- au moins une première chambre (4) comprenant une paroi arrière (3.1) et une paroi avant (3.2), les premières chambres étant prévues à l'intérieur de la pointe (3) de l'endoscope,
- au moins un ballon (5) de type cylindrique qui peut s'allonger dans la direction axiale à travers le colon lors du gonflage, qui présente une entrée (5.2) reliée à une extrémité de l'arbre (2) et une pointe fermée (5.1) disposée dans la première chambre (4), et dans lequel le ballon (5) permet d'avancer la pointe (3) de l'endoscope par pression sur la paroi arrière (3.1) de la pointe (3) de l'endoscope pendant son gonflage.

2. Dispositif médical (1) selon la revendication 1, **caractérisé par** l'arbre (2) à travers lequel de l'air peut passer de manière étanche lorsque de l'air est fourni par son une extrémité non reliée au ballon (5).

3. Dispositif médical (1) selon la revendication 1, **caractérisé par** au moins un mécanisme de rétraction (8) qui est fixé de préférence sur la paroi avant (3.3) à l'intérieur de la première chambre (4) de sorte que la rétraction effectuée pendant le dégonflage du ou des ballons (5) puisse être effectuée d'une manière plus contrôlée et constituée par un arbre (8.1) de préférence sur laquelle est fixée la pointe fermée (5.1) du ballon (5) et autour de laquelle est enroulée la partie dégonflée (5.4) du ballon (5), et un ressort spiralé (8.2) auquel est fixé l'arbre (8.1) afin de fournir le mouvement rotatif nécessaire à ce processus d'enroulement.

4. Dispositif médical (1) selon la revendication 1, **caractérisé par** une chambre séparée, de préférence dans la partie centrale à l'intérieur de la pointe (3) de l'endoscope, pour le matériel (D) placé à la pointe (3) de l'endoscope pour applications endoscopiques.

5. Dispositif médical (1) selon la revendication 1, **caractérisé par** le au moins un ballon (5) qui permet d'avancer la pointe (3) de l'endoscope en appuyant sur la paroi avant (3.3) de la pointe (3) de l'endoscope pendant son gonflage.

6. Dispositif médical (1) selon la revendication 1, **caractérisé par** au moins un trou (3.2) pratiqué sur la pointe (3) de l'endoscope de telle sorte qu'un matériel (D) permettant d'effectuer des procédures médicales puisse être fixé.

7. Dispositif médical (1) selon la revendication 1, **caractérisé par** au moins deux chambres à air (6) qui sont positionnées à l'intérieur de l'arbre (2) de telle sorte qu'il y ait une distance prédéterminée entre elles dans un axe coupant verticalement l'axe où le ballon (5) est placé, et qui permet de déplacer l'arbre (2) dans un axe qui ne peut pas être affecté par les ballons (5) en étant gonflé et dégonflé et permet d'augmenter la distance angulaire qui peut être visualisée via un matériel (D) permettant d'effectuer des procédures médicales.

8. Dispositif médical (1) selon la revendication 1, **caractérisé par** deux ballons (5) dont les entrées (5.2) sont placées sur l'arbre (2) de telle sorte qu'il y ait une distance entre eux et qui permet de modifier la position angulaire de la pointe (3) de l'endoscope et d'avancer la pointe (3) de l'endoscope en même temps qu'ils sont gonflés à différentes tailles en faisant passer l'air hors du patient à l'extrémité de l'arbre (2) à travers un dispositif.

9. Dispositif médical (9) selon la revendication 1, **caractérisé par** des chambres à air (6) qui sont positionnées à l'intérieur de l'arbre (2) dans un axe coupant verticalement l'axe où les ballons (5) sont positionnés, et qui permet de déplacer l'arbre (2) dans un axe qui ne peut pas être affecté par les ballons (5) en étant gonflé et dégonflé par un dispositif et permet d'augmenter la distance angulaire qui peut être visualisée via un matériel (D) permettant d'effectuer des procédures médicales.

10. Dispositif médical (1) selon la revendication 1, **caractérisé par** quatre ballons (5) dont les entrées (5.2) sont placées sur l'arbre (2) de telle sorte qu'elles soient sur deux axes se coupant verticalement et qui permettent de modifier la position angulaire de la pointe (3) de l'endoscope dans deux axes différents et de déplacer la pointe (3) en avant au même moment où ils sont gonflés à différentes tailles en fournissant de l'air à l'extrémité de l'arbre (2) en dehors du patient à travers un dispositif.

11. Dispositif médical (1) selon la revendication 1, **caractérisé par** un conduit de câble (7) qui s'étend de l'axe central de l'arbre (2) vers la pointe (3) de l'endoscope depuis une extrémité de l'arbre (2) à l'extérieur du patient afin d'assurer la communication par câble avec un matériel (D) placé sur la pointe (3) de l'endoscope pour applications endoscopiques.
